# EUROPEAN PATENT APPLICATION

(11) **EP 1 090 991 A1**
(43) Date of publication of application: **11.04.2001**
(21) Application number: 99402371.1
(22) Date of filing: 29.09.1999
(51) Int. Cl.: C12N 15/12, C12N 5/10, C07K 14/705, C07K 16/28, C12Q 1/68, G01N 33/50, G01N 33/566, A61K 31/70, A61K 38/17, A61P 25/00

(54) **Novel nicotinic acetylcholine receptor subunit, its isolation and use**

(71) Applicant: SANOFI-SYNTHELABO, 75013 Paris (FR)
(72) Inventor: Besnard, François, 78220 Viroflay (FR); Charpantier, Eric, 92000 Nanterre (FR); Sgard, Frédéric, 92500 Rueil Malmaison (FR)
(74) Representative: Thouret-Lemaitre, Elisabeth

(57) **Abstract**

HNARA10 polypeptides and polynucleotides and methods for producing such polypeptides by recombinant techniques are disclosed. HNARA10 polypeptides belong to the family of nicotinic acetylcholine receptors. Also disclosed are methods for utilizing HNARA10 polypeptides, polynucleotides and antibodies in the design of protocols for the treatment of various diseases, among others and diagnostic assays for such conditions.

Sequence listing

LISTE DE SEQUENCES

<110> SANOFI-SYNTHELABO

<120> Novel nicotinic acetylcholine receptor subunit, its
isolation and use

<130> HNARA10t

<140>
<141>

<160> 2

<170> PatentIn Ver. 2.1

<210> 1
<211> 1932
<212> ADN
<213> Homo sapiens

<220>
<221> CDS
<222> (43)..(1392)

<400> 1
tcacatccag agacctgccc ccgctcttgc agtgccaggg cc atg ggg ctc cgg
54
Met Gly Leu Arg
1

agc cac cac ctc agc ctg ggc ctt ctg ctt ctg ttt cta ctc cct gca
102
Ser His His Leu Ser Leu Gly Leu Leu Leu Leu Phe Leu Leu Pro Ala
5                   10                  15                  20

gag tgc ctg gga gct gag ggc cgg ctg gct ctc aag ctg ttc cgt gac
150
Glu Cys Leu Gly Ala Glu Gly Arg Leu Ala Leu Lys Leu Phe Arg Asp
                25                  30                  35

ctc ttt gcc aac tac aca agt gcc ctg aga cct gtg gca gac aca gac
198
Leu Phe Ala Asn Tyr Thr Ser Ala Leu Arg Pro Val Ala Asp Thr Asp
            40                  45                  50

cag act ctg aat gtg acc ctg gag gtg aca ctg tcc cag atc atc gat
246
Gln Thr Leu Asn Val Thr Leu Glu Val Thr Leu Ser Gln Ile Ile Asp
        55                  60                  65

atg gat gaa cgg aac cag gtg ctg acc ctg tat ctg tgg ata cgg cag
294
Met Asp Glu Arg Asn Gln Val Leu Thr Leu Tyr Leu Trp Ile Arg Gln
    70                  75                  80

gag tgg aca gat gcc tac cta cga tgg gac ccc aat gcc tat ggt ggc
342
Glu Trp Thr Asp Ala Tyr Leu Arg Trp Asp Pro Asn Ala Tyr Gly Gly
85                  90                  95                  100

390 ctg gat gcc atc cgc atc ccc agc agt ctt gtg tgg cgg cca gac atc
Leu Asp Ala Ile Arg Ile Pro Ser Ser Leu Val Trp Arg Pro Asp Ile
                105                 110                 115

438 gta ctc tat aac aag gcc gac gcg cag cct cca ggt tcc gcc agc acc
Val Leu Tyr Asn Lys Ala Asp Ala Gln Pro Pro Gly Ser Ala Ser Thr
            120                 125                 130

486 aac gtg gtc ctg cgc cac gat ggc gcc gtg cgc tgg gac gcg ccg gcc
Asn Val Val Leu Arg His Asp Gly Ala Val Arg Trp Asp Ala Pro Ala
        135                 140                 145

534 atc acg cgc agc tcg tgc cgc gtg gat gta gca gcc ttc ccg ttc gac
Ile Thr Arg Ser Ser Cys Arg Val Asp Val Ala Ala Phe Pro Phe Asp
    150                 155                 160

582 gcc cag cac tgc ggc ctg acg ttc ggc tcc tgg act cac ggc ggg cac
Ala Gln His Cys Gly Leu Thr Phe Gly Ser Trp Thr His Gly Gly His
165                 170                 175                 180

630 caa ctg gat gtg cgg ccg cgc ggc gct gca gcc agc ctg gcg gac ttc
Gln Leu Asp Val Arg Pro Arg Gly Ala Ala Ala Ser Leu Ala Asp Phe
                185                 190                 195

678 gtg gag aac gtg gag tgg cgc gtg ctg ggc atg ccg gcg cgg cgg cgc
Val Glu Asn Val Glu Trp Arg Val Leu Gly Met Pro Ala Arg Arg Arg
            200                 205                 210

726 gtg ctc acc tac ggc tgc tgc tcc gag ccc tac ccc gac gtc acc ttc
Val Leu Thr Tyr Gly Cys Cys Ser Glu Pro Tyr Pro Asp Val Thr Phe
        215                 220                 225

774 acg ctg ctg ctg cgc cgc cgc gcc gcc gcc tac gtg tgc aac ctg ctg
Thr Leu Leu Leu Arg Arg Arg Ala Ala Ala Tyr Val Cys Asn Leu Leu
    230                 235                 240

822 ctg ccc tgc gtg ctc atc tcg ctg ctt gcg ccg ctc gcc ttc cac ctg
Leu Pro Cys Val Leu Ile Ser Leu Leu Ala Pro Leu Ala Phe His Leu
245                 250                 255                 260

870 cct gcc gac tca ggc gag aag gtg tcg ctg ggc gtc acc gtg ctg ctg
Pro Ala Asp Ser Gly Glu Lys Val Ser Leu Gly Val Thr Val Leu Leu
                265                 270                 275

918 gcg ctc acc gtc ttc cag ttg ctg ctg gcc gag agc atg cca ccg gcc
Ala Leu Thr Val Phe Gln Leu Leu Leu Ala Glu Ser Met Pro Pro Ala
            280                 285                 290

966 gag agc gtg ccg ctc atc ggg aag tat tac atg gcc act atg acc atg
Glu Ser Val Pro Leu Ile Gly Lys Tyr Tyr Met Ala Thr Met Thr Met
        295                 300                 305

1014 gtc aca ttc tca aca gca ctc acc atc ctt atc atg aac ctg cat tac
Val Thr Phe Ser Thr Ala Leu Thr Ile Leu Ile Met Asn Leu His Tyr
    310                 315                 320

1062 tgt ggt ccc agt gtc cgc cca gtg cca gcc tgg gct agg gcc ctc ctg
Cys Gly Pro Ser Val Arg Pro Val Pro Ala Trp Ala Arg Ala Leu Leu
325                 330                 335                 340

1110 ctg gga cac ctg gca cgg ggc ctg tgc gtg cgg gaa aga ggg gag ccc
Leu Gly His Leu Ala Arg Gly Leu Cys Val Arg Glu Arg Gly Glu Pro
                345                 350                 355

1158 tgt ggg cag tcc agg cca cct gag tta tct cct agc ccc cag tcg cct
Cys Gly Gln Ser Arg Pro Pro Glu Leu Ser Pro Ser Pro Gln Ser Pro
            360                 365                 370

1206 gaa gga ggg gct ggc ccc cca gcg ggc cct tgc cac gag cca cga tgt
Glu Gly Gly Ala Gly Pro Pro Ala Gly Pro Cys His Glu Pro Arg Cys
        375                 380                 385

1254 ctg tgc cgc cag gaa gcc cta ctg cac cac gta gcc acc att gcc aat
Leu Cys Arg Gln Glu Ala Leu Leu His His Val Ala Thr Ile Ala Asn
    390                 395                 400

1302 acc ttc cgc agc cac cga gct gcc cag cgc tgc cat gag gac tgg aag
Thr Phe Arg Ser His Arg Ala Ala Gln Arg Cys His Glu Asp Trp Lys
405                 410                 415                 420

1350 cgc ctg gcc cgt gtg atg gac cgc ttc ttc ctg gcc atc ttc ttc tcc
Arg Leu Ala Arg Val Met Asp Arg Phe Phe Leu Ala Ile Phe Phe Ser
                425                 430                 435

1392 atg gcc ctg gtc atg agc ctc ctg gtg ctg gtg cag gcc ctg
Met Ala Leu Val Met Ser Leu Leu Val Leu Val Gln Ala Leu
            440                 445                 450

1452 tgagggctgg gactaagtca cagggatctg ctgcagccac agctcctcca gaaagggaca

1512 gccacggcca agtggttgct ggtctttggg ccagccagtc tctccccact gctcctaaga

1572 tcctgagaca cttgacttca caatccacaa gggagcactc attgtctaca caccctaact

1632 aaaggaagtc cagagcctgc cactccccta attccaaaaa aaagaggaac tctacaaagg

1692 ccaagatcac agagtacagt cttggaggga cagaattgtt tgtgctgggt attggagctc

1752 tcagtgggga gcacatgggt tataatgaga aactgaactg tactgctgca tttcctgtct

tccttcctag gtggctgctt tgcagggctt tggctgttac ctttccctgc tgaggggctc
1812

agggaaaagg gtcggggatt ctcagtcgag tttccagagc aggaggccct acagacattt
1872

cgccccaaat ccctgactca ataaagtaag cgtgtaccta gcaaaaaaaa aaaaaaaaaa
1932

<210> 2
<211> 450
<212> PRT
<213> Homo sapiens

<400> 2
Met Gly Leu Arg Ser His His Leu Ser Leu Gly Leu Leu Leu Leu Phe
1               5                   10                  15

Leu Leu Pro Ala Glu Cys Leu Gly Ala Glu Gly Arg Leu Ala Leu Lys
            20                  25                  30

Leu Phe Arg Asp Leu Phe Ala Asn Tyr Thr Ser Ala Leu Arg Pro Val
        35                  40                  45

Ala Asp Thr Asp Gln Thr Leu Asn Val Thr Leu Glu Val Thr Leu Ser
    50                  55                  60

Gln Ile Ile Asp Met Asp Glu Arg Asn Gln Val Leu Thr Leu Tyr Leu
65                  70                  75                  80

Trp Ile Arg Gln Glu Trp Thr Asp Ala Tyr Leu Arg Trp Asp Pro Asn
                85                  90                  95

Ala Tyr Gly Gly Leu Asp Ala Ile Arg Ile Pro Ser Ser Leu Val Trp
            100                 105                 110

Arg Pro Asp Ile Val Leu Tyr Asn Lys Ala Asp Ala Gln Pro Pro Gly
        115                 120                 125

Ser Ala Ser Thr Asn Val Val Leu Arg His Asp Gly Ala Val Arg Trp
    130                 135                 140

Asp Ala Pro Ala Ile Thr Arg Ser Ser Cys Arg Val Asp Val Ala Ala
145                 150                 155                 160

Phe Pro Phe Asp Ala Gln His Cys Gly Leu Thr Phe Gly Ser Trp Thr
                165                 170                 175

His Gly Gly His Gln Leu Asp Val Arg Pro Arg Gly Ala Ala Ala Ser
            180                 185                 190

Leu Ala Asp Phe Val Glu Asn Val Glu Trp Arg Val Leu Gly Met Pro
        195                 200                 205

Ala Arg Arg Arg Val Leu Thr Tyr Gly Cys Cys Ser Glu Pro Tyr Pro
    210                 215                 220

Asp Val Thr Phe Thr Leu Leu Leu Arg Arg Arg Ala Ala Ala Tyr Val
225                 230                 235                 240

Cys Asn Leu Leu Leu Pro Cys Val Leu Ile Ser Leu Leu Ala Pro Leu
                245                 250                 255

Ala Phe His Leu Pro Ala Asp Ser Gly Glu Lys Val Ser Leu Gly Val
            260                 265                 270

Thr Val Leu Leu Ala Leu Thr Val Phe Gln Leu Leu Leu Ala Glu Ser
        275                 280                 285

Met Pro Pro Ala Glu Ser Val Pro Leu Ile Gly Lys Tyr Tyr Met Ala
    290                 295                 300

Thr Met Thr Met Val Thr Phe Ser Thr Ala Leu Thr Ile Leu Ile Met
305                 310                 315                 320

Asn Leu His Tyr Cys Gly Pro Ser Val Arg Pro Val Pro Ala Trp Ala
                325                 330                 335

Arg Ala Leu Leu Leu Gly His Leu Ala Arg Gly Leu Cys Val Arg Glu
            340                 345                 350

Arg Gly Glu Pro Cys Gly Gln Ser Arg Pro Pro Glu Leu Ser Pro Ser
        355                 360                 365

Pro Gln Ser Pro Glu Gly Gly Ala Gly Pro Pro Ala Gly Pro Cys His
    370                 375                 380

Glu Pro Arg Cys Leu Cys Arg Gln Glu Ala Leu Leu His His Val Ala
385                 390                 395                 400

Thr Ile Ala Asn Thr Phe Arg Ser His Arg Ala Ala Gln Arg Cys His
                405                 410                 415

Glu Asp Trp Lys Arg Leu Ala Arg Val Met Asp Arg Phe Phe Leu Ala
            420                 425                 430

Ile Phe Phe Ser Met Ala Leu Val Met Ser Leu Leu Val Leu Val Gln
        435                 440                 445

Ala Leu
    450

## Description

The invention relates to newly identified polynucleotides, to polypeptides encoded by them and to the use of such polynucleotides and polypeptides. The invention also relates to their production. More particularly, the polynucleotides and polypeptides of the present invention relate to a new member of the nicotinic acetylcholine receptor family, hereinafter referred to as HNARA10. The invention also relates to inhibiting or activating the action of such polynucleotides and polypeptides.

Nicotinic acetylcholine receptors (nAChRs) are members of the ligand-gated ion channel family which also comprises GABA_{A}, glycine and 5-HT₃ receptors (Barnard, E. (1992) *TIBS* 17, 368-374). They are thought to be formed by the association of five homologous subunits orientated around a cation channel, the extracellular N-terminal domain of each subunit determining the natural ligand-binding sites (Galzi, J.-L. & Changeux, J. -P. (1995) *Neuropharmacol.* 34 (6), 563-582). This extracellular domain can, as well as other parts of the receptor, also be the target of many natural and synthetic ligands able to modulate the activity of the receptor (Williams, M. *et al.* (1994) *Drugs News Perspect.* 7, 205-223). These nAChRs form one of the predominant excitatory neurotransmitter receptors on muscles and nerves of the peripheral nervous system and are also expressed in neurons throughout the central nervous system. In addition, they have also been reported in non excitatory cells such as lymphocytes or keratinocytes. nAChRs are associated with a large number of diseases (Lindstrom, J. (1997) *Mol. Neurobiol.* 15 (2), 193-222). Various autoimmune responses to muscle nAChRs can cause muscular paralysis. Mutations in neuronal nAChRs have been shown to cause epilepsy and it has been suggested that genetic defects of some nAChRs may be responsible for schizophrenia. An important loss of nAChRs has also been detected in the brains of patients with Alzheimer's and Parkinson's disease. nAChRs also mediate the addictive effect of nicotine. Furthermore, nicotine has been reported to have a beneficial effect on patients with Tourette's disease and several studies also suggest that nicotinic agonists may have neuroprotective effects. Nicotinic agonists have also analgesic effects and it has been shown that anesthetics can also act on nAChRs. Nicotine can also modulate the heart rate and blood pressure through neuronal nAChRs and it has also been shown to be a mitogenic agent which could promote certain forms of cancer.

This indicates that these receptors have an established, proven history as therapeutic targets. Clearly there is a need for identification and characterization of other receptors which can play a role in preventing, ameliorating or correcting dysfunctions or diseases, including, but not limited to, Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke and appetite disorder, and in producing analgesia or anesthesia.

In one aspect, the invention relates to HNARA10 polypeptides and polynucleotides and to recombinant materials and methods for their production. Another aspect of the invention relates to methods for using such HNARA10 polypeptides and polynucleotides, for example in the treatment of Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke or appetite disorder, and in effecting analgesia or anesthesia, among others. In still another aspect, the invention relates to methods to identify agonists and antagonists using the materials provided by the invention, and treating conditions associated with the use of such compounds. Yet another aspect of the invention relates to diagnostic assays for detecting diseases associated with inappropriate HNARA10 activity or levels.

Figure 1 shows the amino acid sequence encoded by the HNARA10 cDNA. Cleavage of the signal peptide is predicted between amino acid positions 24 and 25 (using SignalP analysis software version 1.1, CBS) and the predicted signal peptide sequence is underlined with a dotted line. Predicted membrane spanning regions (MSR I-IV) (using TMHMM analysis software version 1.0, CBS) are underlined with a solid line. Asterisks indicate cysteine residues at positions 154, 168, 218 and 219 conserved in all nAChR alpha subunits.

Figure 2 shows the alignment of HNARA10 amino acid sequence with a known nAChR, the rat alpha 9 protein sequence, using MEGALIGN software (version 3.13) from DNASTAR with the Jotun-Hein algorithm.

Figure 3 shows a phylogenic tree illustrating the relationship between HNARA10 protein and other vertebrate nAChR subunits. The length of each pair of branches represents the distance between sequence pairs. Alignment was carried out using the CLUSTAL algorithm of MEGALIGN (version 3.13) from DNASTAR.

Figure 4 shows the chromosomal localization (indicated by the arrow) of the human HNARA10 gene as determined by fluorescent *in situ* hybridization.

Figure 5 illustrates the cloning of the full length HNARA10 cDNA.

Figure 6 (a & b) shows the distribution of the HNARA10 mRNA in human tissues as determined by a Master dot blot (Clontech) hybridized with a HNARA10 cDNA probe. In figure 6a, the strong hybridization signal corresponds to human skeletal muscle mRNA. In the signal quantification shown in figure 6b, the signals shown in black are barely above background noise and not significant.

Figure 7 shows the distribution of the HNARA10 mRNA in human tissues as determined by MTN™ blot hybridized with a HNARA10 cDNA probe. The 6.4 kb specifically-labeled transcript is indicated by the arrow.

The following definitions are provided to facilitate understanding of certain terms used frequently herein.

"HNARA10" refers to a polypeptide comprising the amino acid sequence set forth in SEQ ID NO:2, or an allelic variant thereof, as well as other variants as described further below.

"HNARA10 Activity" or "Biological Activity of HNARA10" refers to the metabolic or physiologic function of said HNARA10 including similar activities or improved activities or these activities with decreased undesirable side-effects. Also included are antigenic and immunogenic activities of said HNARA10.

"HNARA10 gene" refers to a polynucleotide comprising the nucleotide sequence set forth in SEQ ID NO:1 or allelic variants thereof and/or their complements as described in the following specification.

"Antibodies" as used herein include polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein. For example, recombinant DNA molecules contained in a vector are considered isolated for the purposes of the present invention. Further examples of isolated nucleic acid molecules include recombinant DNA molecules maintained in heterologous host cells or purified DNA molecules in solution. Isolated RNA molecules include *in vivo* or *in vitro* RNA transcripts of the DNA molecules of the present invention. Isolated nucleic acid molecules according to the invention further include such molecules produced synthetically.

"Polynucleotide" generally refers to any polyribonucleotide or polydeoxyribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons as explained in the present application. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. Thus, "polynucleotide" also encompasses variants of the nucleic acid molecules of the invention which encode portions, analogs or derivatives of the HNARA10 receptors. Variants may occur naturally, such as natural allelic variants. By an "allelic variant" is intended one of several alternate forms of a gene occupying a given locus on a chromosome of an organism. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and molecular properties, 2nd Ed., T. E. Creighton, W. H. Freeman and Company, New York, 1993 and Wold, F, Post-translational Protein Modifications: Perspectives and Prospects, p. 1-12 in Post-translational covalent modification of proteins, B. C. Johnson, Ed., Academic Press, New York, 1983; Seifter *et al.,* "Analysis for protein modifications and non-protein cofactors", *Meth. Enzymol.* (1990) 182:626-646 and Rattan *et al.,* "Protein Synthesis: Post-translational Modifications and Aging", *Ann. NY Acad. Sci.* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring, such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g. : Computational Molecular Biology, Lesk, A.M., ed., Oxford University Press, New York, 1988; Biocomputing Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991. While there exists a number of methods to measure identity between two polynucleotide or polypeptide sequences, the term "identity" is well known to skilled artisans (Carillo, H., and Lipton, D., *SIAM J. Applied Math.* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J. Applied Math.* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al., J. Molec. Biol.* (1990) 215:403).

As an illustration, by a polynucleotide having a nucleotide sequence having at least, for example, 95% "identity" to a reference nucleotide sequence of SEQ ID NO: 1 is intended a polynucleotide in which the nucleotide sequence is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended a polypeptide having an amino acid sequence identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

In one aspect, the present invention relates to isolated HNARA10 polypeptides. The HNARA10 polypeptides include the polypeptide of SEQ ID NO:2; as well as polypeptides comprising the amino acid sequence of SEQ ID NO:2; and polypeptides comprising an amino acid sequence which has at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within HNARA10 polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% are highly preferred. Preferably HNARA10 polypeptides exhibit at least one biological activity of HNARA10.

It will be recognized that some amino acids of the HNARA10 polypeptides can be varied without significant effect on the structure or function of the protein. If such differences are contemplated, it should be remembered that there will be critical areas on the protein which determine activity.

Thus, the invention further provides variants of the HNARA10 polypeptides which show substantial HNARA10 polypeptide activity or which include regions of HNARA10 protein such as the protein portions discussed below. Such mutants include HNARA10 polypeptides in which deletions, insertions, type substitutions, inversions and/or repeats have occurred. Guidance concerning with amino acid changes which are likely to be phenotypically silent can be found in Bowie, J. U. et al, "Deciphering the Message in Protein Sequences : Tolerance to Amino Acid Substitutions", *Science* 247 : 1306-1310 (1990).

Thus, the fragment, derivative or analog of the polypeptide of SEQ ID NO:2, or that encoded by the deposited cDNA as discussed below, may be :
- one in which one or more of the amino acid residues are substituted with a conserved or non-conserved amino acid residue, preferably a conserved amino acid residue, and such substituted amino acid residue may or may not be one encoded by the genetic code, or
- one in which one or more of the amino acid residues includes a substituent group, or
- one in which the mature polypeptide is fused with another compound, such as a compound for increasing the half-life of the polypeptide (for example, polyethylene glycol), or
- one in which additional amino acids are fused to the mature polypeptide, such as the IgG Fc fusion region peptide or leader or secretory sequence which is employed for purification of the mature polypeptide or a pro-protein sequence. Such fragments, derivatives and analogs are deemed to be within the scope of the invention.

Of particular interest are substitutions of charged amino acids with another charged amino acid and with neutral or negatively charged amino acids. The latter results in proteins with reduced positive charge to improve the characteristics of the HNARA10 protein, for example for prevention of aggregation.

As indicated, changes are preferably of a minor nature, such as conservative amino acid substitutions that do not significantly affect the binding or activity of the protein, such as those indicated hereafter.

| | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic acid |
| | Glutamic acid |
| Small | Alanine |
| | Serine |
| | Threonine |
| | Methionine |
| | Glycine |

Generally, the number of amino acid substitutions will not be more than 50.

Amino acids in the HNARA10 polypeptide of the present invention that are essential for function can be identified by methods known in the art, such as site-directed mutagenesis or alanine-scanning mutagenesis (Cunnigham and Wells, *Science* 244 : 1081-1085 (1989)). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis, e.g. crystallization, nuclear magnetic resonance or photoaffinity labeling (Smith et al., *J*. *Mol. Biol.* 224 : 899-904 (1992); de Vos et al., Science 255 : 306-312 (1992)).

As to the selection of peptides or polypeptides bearing an antigenic epitope, it is well known that relatively short synthetic sequences that mimic part of a protein sequence are routinely capable of eliciting an antiserum that reacts with the partially mimicked protein. See for instance Sutcliffe, J. G., Shinnick, T. M. , Green, N. and Learner, R. A., Antibodies that React with Predetermined Sites on Proteins, *Science* 219 : 660-666 (1983). Peptides capable of eliciting protein-reactive sera are frequently represented in the primary sequence of a protein, can be characterized by a set of simple chemical rules, and are confined neither to immunodominant regions of intact proteins (i.e. immunogenic epitopes) nor to the amino or carboxyl terminals.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the invention preferably contain a sequence of at least seven, more preferably at least nine and most preferably between about 15 to about 50 amino acids contained within the amino acid sequence of a polypeptide according to the invention.

For instance, antigenic polypeptides or peptides that can be used to generate HNARA10 specific antibodies comprise : a polypeptide comprising amino acid residues from about 84 to about 101 of SEQ ID NO:2, a polypeptide comprising amino acid residues from about 120 to about 158 of SEQ ID NO:2, a polypeptide comprising amino acid residues from about 177 to about 196 of SEQ ID NO:2, a polypeptide comprising amino acid residues from about 207 to about 226 of SEQ ID NO:2, a polypeptide comprising amino acid residues from about 348 to about 395 of SEQ ID NO:2, a polypeptide comprising amino acid residues from about 405 to about 427 of SEQ ID NO:2. As indicated above, it is believed that the above polypeptide fragments are antigenic regions of the HNARA10 polypeptide as determined using Protean (version 3.16) from DNASTAR. In particular, polypeptides comprising amino acid residues from 179 to 196 and amino acid residues from 357 to 374 of SEQ ID NO:2 were successfully used to raise polyclonal antibodies in rabbit which are able to recognize HNARA10 protein expressed transiently in HEK 293 cells.

The epitope-bearing peptides and polypeptides of the invention can be prepared by any conventional means, for instance by the method described in document US Patent n° 4,631,211.

HNARA10 polypeptides of the present invention and the epitope-bearing fragments thereof can be combined with parts of the constant domain of immunoglobulins (IgG), resulting in chimeric polypeptides. These fusion proteins facilitate the purification and generally show an increased half life *in vivo.* See for instance Traunecker et al., *Nature* 331 : 84-86 (1988). Fusion proteins that have a disulfide-linked dimeric structure due to the IgG part can also be more efficient in binding and neutralizing other molecules than the monomeric HNARA10 protein or protein fragment thereof (Fountoulakis et al., *J. Biochem.* 270 : 3958-3964 (1995)).

The HNARA10 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the HNARA10 polypeptides or variants, mutated forms, derivatives thereof as here above mentioned are also included in the invention. A fragment is a polypeptide having an amino acid sequence that entirely is the same as part, but not all, of the amino acid sequence of the aforementioned HNARA10 polypeptides. As with HNARA10 polypeptides, fragments may be "free-standing," or comprised within a larger polypeptide of which they form a part or region, most preferably as a single continuous region. Representative examples of polypeptide fragments of the invention, include, for example, fragments from about amino acid number 1 to about 235, from about 236 to about 450, from about 321 to about 428 of HNARA10 polypeptide shown in SEQ ID NO:2. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of HNARA10 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate HNARA10 activity, including those with a similar activity or an improved activity, or with a decreased undesirable activity. Also included are those that are antigenic or immunogenic in an animal, especially in a human.

Preferably, all of these polypeptide fragments retain the biological activity of HNARA10, including antigenic activity.

The HNARA10 polypeptides of the invention can be prepared in any suitable manner. Such polypeptides include isolated naturally occurring polypeptides, recombinantly produced polypeptides, or polypeptides produced by a combination of these methods.

In one embodiment, the HNARA10 polypeptides of the invention can be prepared by chemical synthesis, for example by using non natural and/or modified amino acids. Thus, it can be advantageous to use non natural amino acids, for example D-amino acids, to enhance life time of the polypeptides, or analogs of amino acids, for example sulfurized forms of amino acids.

Methods for preparing such polypeptides are known in the art.

For example, chemical synthesis of the polypeptides of the invention can be carried out by using a solid phase process. In such a method, the C-terminal amino acid is fixed onto an inert solid support et comprises protective groups (e.g. BOC or FMOC) of the alpha-amino moiety. At the end of this step, the protective group is removed and a second amino acid, optionally protected in a similar manner, is fixed. The N-terminal protective groups are removed after each one amino acid is fixed, protective groups possibly present on the lateral chains being conserved. When the peptide chain is synthesized, the peptide is cleaved from the support and the remaining protective groups are removed. Coupling can be carried out by using N,N'-diisopropyl-carbodiimine (DIC), among others, in a solvent of DMF or DCM. This solid phase synthesis is described for example in Stewart J. M. et al., Solid Phase Peptides Synthesis. Pierce Chem. Company, Rockford, 111, 2^{nd} Ed., (1984).

Other polypeptides according to the invention comprise the amino acid sequence of the HNARA10 polynucleotide which possesses the complete amino sequence encoded by the cDNA clone contained in ATCC deposit number PTA-315 as discussed below, as well as polypeptides which comprise the amino acid sequence of the mature HNARA10 polynucleotide which possesses the complete amino sequence encoded by the cDNA clone contained in ATCC deposit number PTA-315.

In another aspect, the invention provides a peptide or polypeptide comprising an epitope-bearing portion of a polypeptide of the invention. The epitope of this polypeptide portion is an immunogenic or antigenic epitope of a polypeptide described in the present invention. An immunogenic epitope is defined as a part of a protein that elicits an antibody response when the whole protein is the immunogen. On the other hand, a region of a protein molecule to which an antibody can bind is defined as an antigenic epitope.

Another aspect of the invention relates to HNARA10 polynucleotides.

Unless otherwise indicated, all nucleotide sequences determined by sequencing a DNA molecule herein were determined by using an automated DNA sequencer, and all amino acid sequences of polypeptides encoded by DNA sequences determined herein were predicted by translation of a DNA sequence determined as above. Therefore, as is known in the art for any DNA sequence determined by this approach, any nucleotide sequence may contain some errors. Nucleotide sequences determined by automation are typically at least about 90 % identical, more typically at least about 95 % to about 99.9 % identical to the actual nucleotide sequence of the sequenced DNA molecule.

HNARA10 polynucleotides include isolated polynucleotides which encode the HNARA10 polypeptides and fragments, and polynucleotides closely related thereto, e.g. variants, derivatives, mutated forms. More specifically HNARA10 polynucleotides of the invention include a polynucleotide comprising the nucleotide sequence set forth in SEQ ID NO:1 encoding a HNARA10 polypeptide of SEQ ID NO: 2, and a polynucleotide having the particular sequence of SEQ ID NO:1. HNARA10 polynucleotides further include a polynucleotide comprising a nucleotide sequence that has at least 80% identity to a nucleotide sequence encoding the HNARA10 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under HNARA10 polynucleotides are nucleotide sequences which have sufficient identity to a nucleotide sequence contained in SEQ ID NO:1 or contained in the cDNA insert of the plasmid deposited with the ATCC Deposit number PTA-315 to hybridize under conditions useable for amplification or for use as a probe or marker. Moreover, HNARA10 polynucleotides include a nucleotide sequence having at least 80% identity to a nucleotide sequence encoding the HNARA10 polypeptide, either in a mature form or not, expressed by the cDNA insert deposited at the ATCC with Deposit Number PTA-315 as discussed below, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the HNARA10 polynucleotides according to the invention.

A deposit containing a human HNARA10 cDNA has been deposited with the American Type Culture Collection (ATCC), 10801 University Blvd, Manassas, VA 20110-2209, USA, on July 7, 1999, and assigned ATCC Deposit Number PTA-315. The deposited material (clone) is pCDNA3 that further contains the full length HNARA10 cDNA, referred to as Human plasmid HNARA10 upon deposit. The cDNA insert is within BamH I and Xba I sites in the vector. The nucleotide sequence of the polynucleotide contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

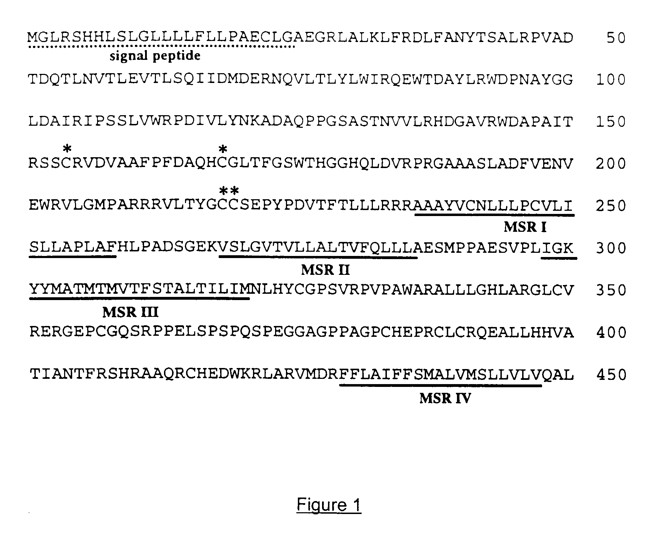

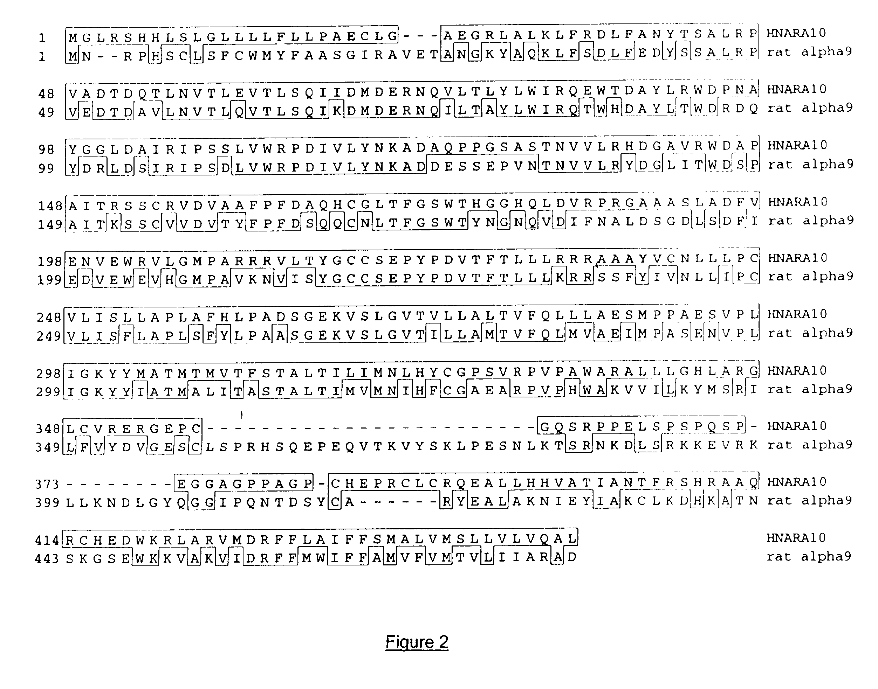

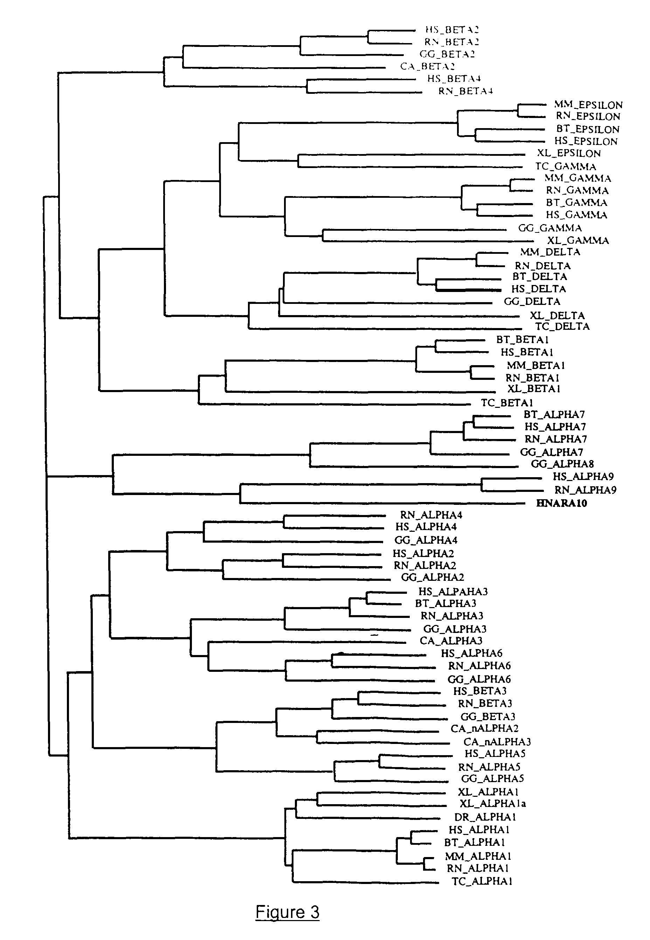

HNARA10 of the invention is structurally related to other proteins of the nicotinic acetylcholine receptor family, as shown by the results of sequencing the cDNA of SEQ ID NO:1 encoding human HNARA10. The cDNA sequence of SEQ ID NO:1 contains an open reading frame (nucleotide numbers 43 to 1392) encoding a polypeptide of about 450 amino acid residues of SEQ ID NO:2, with a predicted leader sequence of about 24 amino acid residues and a deduced molecular weight of about 49.7 kDa. Amino acid sequence analysis (using SignalP version 1.1 and TMHMM prediction software version 1.0, Centre for Biological Sequence analysis) shows that the HNARA10 predicted protein sequence comprises all the typical features of nicotinic acetylcholine receptor alpha subunits: a predicted signal peptide, four potential transmembrane domains, two cysteine residues separated by 13 amino acids and a pair of adjacent cysteine residues in the predicted extracellular N-terminal region (Figure 1). Amino acid sequence of SEQ ID NO:2 has about 57% identity (using MEGALIGN version 3.13 with the Jotun-Hein algorithm from DNASTAR) in 1-450 amino acid residues with the rat nicotinic acetylcholine receptor alpha 9 subunit (Swissprot accession number P43144) (Figure 2). Nucleotide sequence of SEQ ID NO:1 has about 62% identity (using MEGALIGN version 3.13 with the Jotun-Hein algorithm from DNASTAR) in 43-1392 nucleotide residues with the rat nicotinic acetylcholine receptor alpha 9 subunit coding sequence (Genbank accession number U12336). An alignment of the HNARA10 amino acid sequence with that of other known vertebrate nicotinic acetylcholine receptor subunits (using MEGALIGN version 3.13 with the CLUSTAL algorithm from DNASTAR) shows that the HNARA10 protein is most related to the homomeric nicotinic receptor subunits subgroup which includes alpha 7, alpha 8 and alpha 9 subunits (Figure 3).

The polynucleotides of the present invention encoding HNARA10 polypeptide may be obtained using standard cloning and screening, from a cDNA library derived from mRNA in cells of human skeletal muscle, stomach antrum or intestinal duodenum, using the expressed sequence tag (EST) analysis (Adams, M.D., *et al. Science* (1991) 252:1651-1656; Adams, M. D. *et al., Nature,* (1992) 355:632-634; Adams, M. D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding HNARA10 polypeptide of SEQ ID NO:2 may be identical to the polypeptide encoding sequence contained in SEQ ID NO:1 (nucleotide numbers 43 to 1392), or it may be a different sequence, which as a result of the redundancy (degeneracy) of the genetic code, also encodes the polypeptide of SEQ ID NO:2.

As indicated, the invention also provides the mature form of the HNARA10 receptor of the present invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species of the protein. Further, it has long been known that the cleavage specificity of a secreted protein is ultimately determined by the primary structure of the complete protein, i.e. its amino acid sequence. Therefore, the invention provides a nucleotide sequence encoding the mature HNARA10 polypeptide having the amino acid sequence encoded by the cDNA clone contained in the host identified as ATCC Deposit PTA-315. By the mature HNARA10 polypeptide having the amino acid sequence encoded by the cDNA clone contained in the host identified as ATCC Deposit PTA-315 is meant the mature form of the HNARA10 polypeptide produced by expression in a mammalian cell of the complete open reading frame encoded by the human DNA sequence of the clone contained in the vector in the deposited host. As indicated, the mature HNARA10 polypeptide having the amino acid sequence encoded by the cDNA clone contained in ATCC Deposit PTA-315 may or may not differ form the predicted "mature" HNARA10 protein represented in SEQ ID NO:2, depending on the accuracy of the predicted cleavage site based on computer analysis.

Methods for predicting whether a protein has a secretory leader as well as the cleavage site of this leader sequence are available. For instance, the methods of McGeoch (*Virus Res.* 3:271-286 (1985)) and von Heinje *(Nucleic Acids Res.* 14:4683-4690 (1986)) can be used.

When the polynucleotides of the invention are used for the recombinant production of HNARA10 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) or in the pTracer™ vector (Invitrogen, Inc) and described in Gentz *et al., Proc. Natl. Acad. Sci. USA* (1989) 86:821-824, or is an HA or V5 epitope tag. The polynucleotide may also contain noncoding 5' and 3' sequences, such as transcribed, non-translated sequences, splicing and polyadenylation signals, ribosome binding sites and sequences that stabilize mRNA.

Further preferred embodiments are polynucleotides encoding HNARA10 variants comprising the amino acid sequence of HNARA10 polypeptide of SEQ ID NO:2 in which several, 5-10, 1-5, 1-3, 1-2 or 1 amino acid residues are substituted, deleted or added, in any combination.

The invention is further directed to fragments of the isolated nucleic acid molecules described herein. By a fragment of an isolated nucleic acid molecule having the nucleotide sequence of the deposited cDNA or the nucleotide molecule having the nucleotide sequence shown in SEQ ID NO:1 is intended fragments of at least about 15 nucleotides, and preferably of at least about 20 nucleotides, more preferably of at least about 30 nucleotides, even more preferably of at least about 40 nucleotides, which are useful as diagnostic probes and primers. Of course, larger fragments of about 50-1500 nucleotides in length are also useful according to the present invention, as are fragments corresponding to most, if not all, of the nucleotide sequence of the deposited cDNA or as shown in SEQ ID NO:1. By a fragment of at least 20 nucleotides in length, for example, are intended fragments which include 20 or more contiguous bases from the nucleotide sequence of the deposited cDNA or from the sequence shown in SEQ ID NO:1.

In a first aspect, preferred nucleic acid fragments according to the invention comprise those which can be used as nucleic probes.

For example, a nucleic probe according to the invention comprises the nucleotide sequence located between nucleotide number 998 and nucleotide number 1366 of the sequence set forth in SEQ ID NO:1.

Another nucleic probe according to the invention comprises the nucleotide sequence located between nucleotide number 1396 and nucleotide number 1914 of the sequence set forth in SEQ ID NO:1.

In a second aspect, preferred nucleic acid fragments according to the invention comprise a nucleotide sequence encoding for an antigenic region of HNARA10.

For example, preferred nucleic acid fragments according to the invention comprise nucleic acid molecules which encode a polypeptide comprising amino acid residues from about 84 to about 101 of the amino acid sequence shown in SEQ ID NO:2, nucleic acid molecules which encode a polypeptide comprising amino acid residues from about 120 to about 158 of the amino acid sequence of SEQ ID NO:2, nucleic acid molecules which encode a polypeptide comprising amino acid residues from about 177 to about 196 of the amino acid sequence of SEQ ID NO:2, nucleic acid molecules which encode a polypeptide comprising amino acid residues from about 207 to about 226 of the amino acid sequence of SEQ ID NO:2, nucleic acid molecules which encode a polypeptide comprising amino acid residues from about 348 to about 395 of the amino acid sequence of SEQ ID NO:2, nucleic acid molecules which encode a polypeptide comprising amino acid residues from about 405 to about 427 of the amino acid sequence of SEQ ID NO:2, nucleic acid molecules which encode a polypeptide comprising amino acid residues from about 179 to about 196 of the amino acid sequence of SEQ ID NO:2, nucleic acid molecules which encode a polypeptide comprising amino acid residues from about 357 to about 374 of the amino acid sequence of SEQ ID NO:2.

The invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means that hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number PTA-315 or a fragment thereof, may be used as hybridization probes for cDNA and genomic DNA, to isolate full-length cDNAs and genomic clones encoding HNARA10 and to isolate cDNA and genomic clones of other genes that have a high sequence similarity to the HNARA10 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, obtaining a polynucleotide encoding HNARA10 polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

The present invention also relates to recombinant vectors which comprise a polynucleotide or polynucleotides according to the invention and expression system capable of producing the HNARA10 polypeptide when said expression system is present in a compatible host cell, host cells which are genetically engineered with vectors of the invention, and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *Basic Methods in Molecular Biology* (1986) and Sambrook et al., *Molecular Cloning : a Laboratory Manual,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

The expression vectors will preferably include at least one selectable marker. Such markers include dihydrofolate reductase or neomycin resistance for eukaryotic cell culture and tetracycline or ampicillin resistance genes for culturing *E. coli* and other bacteria.

The DNA insert should preferably be linked to an appropriate promoter, such as the bacterial phage lambda PL and RP, T3 and T7, *E. coli lac, trp* and *tac* promoters, the eukaryotic SV40 early and late promoters, CMV immediate early promoters, eukaryotic promoters from retroviral LTRs, such as those of the Rous Sarcoma Virus (RSV) to name a few. Other suitable promoters are available for the skilled artisan.

The expression constructs will further contain sites for transcription initiation, termination and, in the transcribed region, a ribosome binding site for translation. The coding region of the mature transcripts expressed by the constructs will preferably include a translation initiating at the beginning and a termination codon (UAA, AGA or UAG) appropriately positioned at the end of the polypeptide to be translated.

Representative examples of appropriate hosts comprise bacterial cells, such as streptococci, staphylococci, *E. coli, Streptomyces, Salmonella typhimurium* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. Generally, any system or vector suitable to maintain, propagate or express polynucleotides to produce a polypeptide in a host may be used.

Among vectors preferred for use in bacteria are pGE70, pGE60 and pG-9, available form Qiagen, Phagescript vectors, pBS vectors, Bluescript vectors, pNH8A, pNH16a, pNH18A, pNH46A, available from Stratagene, ptrc99a, pKK223-3, pDR540, pRIT5 available from Pharmacia.

Among preferred eukaryotic vectors are pWLNEO, pSV2CAT, pOG44, pXT1 and pSG available from Stratagene, pSVK3, pBPV, pMSG and pSVL available from Pharmacia, pcDNA3, pcDNA3.1 and pTracer™ available from Invitrogene. Other suitable vectors will be readily apparent to the skilled in the art.

The appropriate nucleotide sequence may be inserted into an expression system by any of a variety of well-known and routine techniques, such as, for example, those set forth in Sambrook *et al., Molecular Cloning, a Laboratory Manual* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the HNARA10 polypeptide is to be expressed for use in screening assays, generally, it is preferred that the polypeptide be produced at the surface of the cell. In this event, the cells may be harvested prior to use in the screening assay. If HNARA10 polypeptide is secreted into the medium, the medium can be recovered in order to recover and purify the polypeptide; if produced intracellularly, the cells must first be lysed before the polypeptide is recovered.

HNARA10 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region form immunoglobulin that is useful to solubilize proteins. For example, EP-A-0 464 533 discloses fusion proteins comprising various portions of constant region of immunoglobulin molecules together with another human protein or part thereof. In many cases, the Fc part in a fusion protein is advantageous for use in therapy and diagnosis. On the other hand, for some uses, it would be desirable to delete the Fc part after the fusion protein has been expressed, detected and purified. For instance, it is the case when Fc portion proves to be a hindrance to use in therapy and diagnosis, for example when the fusion protein is to be used as antigen for immunizations.

It is believed that mammals with certain diseases or susceptibility to certain diseases express significantly altered, enhanced or diminished levels of the HNARA10 protein and mRNA encoding the HNARA10 protein, when compared to a corresponding "standard" mammal, i.e. a mammal of the same species not having or not presenting a susceptibility to the disease. Further, it is believed that significantly altered, enhanced or diminished levels of the HNARA10 protein can be detected in certain body tissues (e.g. bone marrow, cerebellum, brain, midbrain, spinal cord, nerve endings, retina, breast, pituitary, heart, lung, skeletal muscle, kidney, pancreas, intestine, stomach) from mammals with the disease or presenting a susceptibility to the disease when compared to tissues from mammals of the same species not having or not presenting a susceptibility to the disease. Thus, the invention provides a diagnostic method which involves assaying the expression level of the gene encoding a HNARA10 receptor of the present invention in mammalian cells or body fluid and comparing the gene expression level with a standard HNARA10 receptor expression level, whereby an altered, enhanced or diminished expression level of the gene with respect to the standard is indicative of the disease or of a susceptibility to the disease. Preferred mammals include monkeys, apes, cats, dogs, cows, pigs, horses, rabbits and humans.

Diseases which can be diagnosed include but are not limited to Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke or appetite disorder.

By "assaying the expression level of the gene encoding a HNARA10 receptor" is intended qualitatively or quantitatively measuring or estimating the level of a HNARA10 protein or the level of the mRNA encoding a HNARA10 receptor in a first biological sample either directly (e.g. by determining or estimating absolute protein level or mRNA level) or relatively (e.g. by comparing with the HNARA10 protein level or mRNA level in a second biological sample).

Preferably, the HNARA10 protein level or mRNA level in the first biological sample is measured or estimated and compared with a standard HNARA10 protein level or mRNA level, the standard being taken from a second biological sample obtained from an individual not having (nor presenting a susceptibility) the disease. As will be appreciated, once a standard HNARA10 protein level or mRNA level is known, it can be used repeatedly as a standard for comparison.

By "biological sample" is intended any biological sample, either tissue sample or body fluid, obtained from an individual, cell line, tissue culture, or other source which may contain HNARA10 protein or mRNA.

Total cellular RNA can be isolated from a biological sample using the single-step guanidinium-thiocyanate-phenol-chloroform method described in Chomczynski and Sacchi, *Anal. Biochem.* 162 : 156-159 (1987). Levels of mRNA encoding the HNARA10 receptor are then assayed using any appropriate method. These include Northern Blot analysis (Harada et al., *Cell* 63 : 303-312 (1990)), S1 nuclease mapping (Fujita et al., *Cell* 49 : 357-367 (1987)), the polymerase chain reaction (PCR), reverse transcription in combination with the polymerase chain reaction (RT-PCR) (Fujita et al., *Cell* 49 : 357-367 (1987)), and reverse transcription in combination with the ligase chain reaction (RT-LCR).

Assaying HNARA10 protein levels in a biological sample can occur using antibody-based techniques. For example, HNARA10 protein expression in tissues can be studied with classical immunohistological methods (Jalkanen, M., et al., *J. Cell. Biol.* 101 : 976-985 (1985); Jalkanen, M., et al., *J. Cell. Biol. 105 : 3087-3096 (1987)).* Other antibody-based methods useful for detecting HNARA10 receptor gene expression include immunoassays, such as the enzyme linked immunosorbent assay (ELISA) and the radioimmunoassay (RIA).

Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine and biotin.

In addition, the invention provides a diagnostic assays for diagnosing or determining a susceptibility to, among others, Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke, appetite disorder through detection of mutation in the HNARA10 gene by the methods described below.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HNARA10 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al*, *Proc. Natl. Acad. Sci. USA* (1985) 85: 4397-4401. In another embodiment, an array of oligonucleotides probes comprising HNARA10 nucleotide sequence or fragments thereof can be constructed to conduct efficient screening of e.g., genetic mutations. Array technology methods are well known and have general applicability. They can be used to address a variety of questions in molecular genetics including gene expression, genetic linkage, and genetic variability. (See for example: M.Chee et al., *Science,* Vol 274, pp 610-613 (1996)).

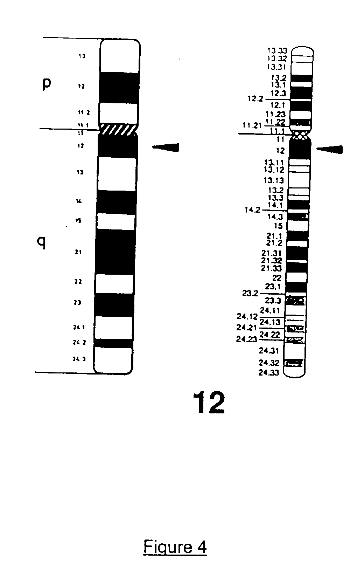

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. In this instance, the HNARA10 gene was localized by fluorescence *in situ* hybridization (FISH) on chromosome 12 at a position immediately adjacent to the centromere on the long arm of the chromosome, corresponding to band 12q12 (Figure 4). Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes)

The differences in the cDNA or genomic sequence between affected and unaffected individuals can also be determined. If a mutation is observed in some or all of the affected individuals but not in any normal individuals, then the mutation is likely to be the causative agent of the disease.

The polypeptides of the invention or their fragments or analogs thereof, or cells expressing them can also be used as immunogens to produce antibodies or fragments of antibodies immunospecific for the HNARA10 polypeptides. The term "immunospecific" means that the antibodies have substantial greater affinity for the polypeptides of the invention than their affinity for other related polypeptides in the prior art.

Antibodies generated against the HNARA10 polypeptides can be obtained by administering the polypeptides or epitope-bearing fragments, analogs or cells to an animal, preferably a non human, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* Monoclonal Antibodies and Cancer Therapy, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against HNARA10 polypeptides may also be employed to treat or to diagnose Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke, appetite disorder, among others.

Another aspect of the invention relates to a method for inducing an immunological response in a mammal which comprises inoculating the mammal with HNARA10 polypeptide, or a fragment thereof, adequate to produce antibody and/or T cell immune response to protect said animal from Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke, appetite disorder, among others. Yet another aspect of the invention relates to a method of inducing immunological response in a mammal which comprises, delivering HNARA10 polypeptide via a vector directing expression of HNARA10 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a HNARA10 polypeptide wherein the composition comprises a HNARA10 polypeptide or HNARA10 gene. The vaccine formulation may further comprise a suitable carrier. Since HNARA10 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

The HNARA10 polypeptide of the present invention may be employed in a screening process for candidate compounds which bind to the receptor and which, directly or indirectly, activate (agonists) or inhibit activation (antagonists) of the receptor polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al.*, *Current Protocols in Immunology* 1 (2):Chapter 5 (1991).

The choice of the candidate compounds may be based on similarity with known agonists or antagonists of the receptor family. In particular, known antagonists or agonists of the nicotinic receptor family may be used to determine candidate compounds to be screened.

Such known compounds include, but are not limited to, (S)-Nicotine, and its homologs Anabasine et Anabaseine, in addition to Cytisine, Epibatidine, Anatoxin-a, lobeline, and other natural products known to activate nicotinic receptor subtypes.

In addition synthetic analogs have been described in the literature as having agonist properties at nicotinic subtypes. These include ABT-418, SIB-1508Y, SIB1563, RJ-2403 or trans-metanicotine, A-85390, ABT-594, and many others.

Known antagonists of nicotinic subtypes include mecamylamine, dihydro-beta-erythroidine, alpha-conotoxins. Of particular interest are compounds which are known to interact with nicotinic receptors which are known to be composed of a homomeric combination of nicotinic receptor alpha subunits such as alpha 7. Known agonists for this subtype are GTS-21, AR-R1777, whilst known antagonists are alpha-bungarotoxin, methyllycaconitine and alpha-conotoxin Iml.

Similarity of the candidate compounds with compounds known to activate or inhibit activation of the nicotinic acetylcholine receptor family may be structural or functional, or both. For example, candidate compounds to be screened may be chosen starting from their chemical resemblance with known compounds. Other candidate compounds may be chosen by taking into account their functional relationship with the nicotinic acetylcholine receptor family.

Other candidate compounds can be chosen among compounds known to be involved in a large number of biological processes and can be selected for screening solely on this basis.

HNARA10 polypeptides are responsible for many biological functions, including many pathologies. Accordingly, it is desirous to find compounds and drugs which stimulate HNARA10 on the one hand or which can inhibit the function of HNARA10 on the other hand. In general, agonists are employed for therapeutic and prophylactic purposes for such conditions as Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke, appetite disorder. Antagonists may be employed for a variety of therapeutic and prophylactic purposes for such conditions as Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke or appetite disorder.

In general, such screening procedures involve producing appropriate cells which express the receptor polypeptide of the present invention on the surface thereof. Such cells include cells from mammals, yeast, *Drosophila* or *E. coli.* Cells expressing the receptor (or cell membrane containing the expressed receptor) are then contacted with a test compound to observe binding, stimulation or inhibition of a functional response.

The assays may simply test the binding of a candidate compound, wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Examples of potential HNARA10 antagonists include antibodies or, in some cases, oligonucleotides or proteins which are closely related to the ligand of the HNARA10, e.g., a fragment of the ligand, or small molecules which bind to the receptor but do not elicit a response, so that the activity of the receptor is prevented.

The invention provides methods of treating abnormal conditions related to both an over-activity or an insufficient activity of HNARA10.

In a first aspect, the invention provides a method of treating an individual in need of an altered, diminished or inhibited level of HNARA10 activity or expression, comprising administering to such an individual a pharmaceutical composition comprising an effective amount of an active ingredient capable of altering or diminishing the HNARA10 activity.

In that case, several approaches are available. One approach comprises administering as active ingredient an inhibitor compound (antagonist) as above described along, with a pharmaceutically acceptable carrier, in an amount effective to inhibit activation by blocking binding of ligands to the HNARA10, or by inhibiting a second signal, and thereby alleviating the abnormal condition.

In another approach, soluble forms of HNARA10 polypeptides still capable of binding the ligand in competition with endogenous HNARA10 may be administered as active ingredient. Typical embodiments of such competitors comprise fragments of the HNARA10 polypeptide.

In still another approach, expression of the gene encoding endogenous HNARA10 can be inhibited using expression blocking techniques. Such known techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al.*, *Nucleic Acids Res.* (1979) 6:3073; Cooney *et al., Science* (1988) 241:456; Dervan *et al., Science* (1991) 251:1360. These oligomers can be administered *per se* or the relevant oligomers can be expressed *in vivo.*

In a second aspect, the invention provides a method of treating an individual in need of an increased level of HNARA10 activity or expression, comprising administering to such an individual a pharmaceutical composition comprising an effective amount of an active ingredient capable of increasing the HNARA10 activity.

In that case, several approaches are also available. One approach comprises administering as active ingredient a therapeutically effective amount of a compound which activates HNARA10, i.e., an agonist as described above, in combination with a pharmaceutically acceptable carrier, to thereby alleviate the abnormal condition.

Alternatively, gene therapy may be employed to effect the endogenous production of HNARA10 by the relevant cells in the subject. For example, a polynucleotide of the invention may be engineered for expression in a replication defective retroviral vector, as discussed above. The polynucleotide constitutes in that case the active ingredient. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, Gene Therapy and other Molecular Genetic-based Therapeutic Approaches, (and references cited therein) in *Human Molecular Genetics,* T Strachan and A P Read, BIOS Scientific Publishers Ltd (1996).

In another aspect, the invention provides pharmaceutical compositions for preventing and/or treating in a subject a disease or susceptibility to a disease related to activity of HNARA10.

Pharmaceutical compositions of the invention are intended, but not limited to, the treatment and/or prevention of Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke, appetite disorder, among others.

In a first aspect, the pharmaceutical compositions of the invention for preventing and/or treating in a subject a disease or susceptibility to a disease related to over-activity or over-expression of HNARA10 may comprise a therapeutically effective amount of at least an antagonist of HNARA10 as defined above, at least a nucleic acid molecule that inhibits the expression of the nucleotide sequence encoding said receptor (antisense sequence) and/or at least a peptide which competes with said receptor, as discussed above.

In a second aspect, the pharmaceutical compositions of the invention for preventing and/or treating in a subject a disease or susceptibility to a disease related to under-activity or under-expression of HNARA10 may comprise a therapeutically effective amount of at least an agonist of HNARA10 as defined above, at least a polynucleotide according to the invention and/or at least a nucleotide sequence according to the invention able to express *in vivo* a HNARA10 polypeptide of the invention.

The pharmaceutical compositions in which at least a polypeptide according to the invention is used may comprise the recombinant vectors of the invention, able to express *in vivo* a polypeptide according to the invention. Polypeptides according to the invention may be also used "naked", i.e. without being inserted into a vector. It has been shown in effect that some DNA or RNA sequences can be expressed in some tissues without the aid of an expression vector.

Pharmaceutical compositions according to the invention, for example those which comprise polypeptides of the invention, such as the soluble form of HNARA10 polypeptides, agonists and antagonists or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, intrasternal, intraarterial or intraperitoneal, can be used. Alternative means for systemic administration include transmucosal and transdermal administration using penetrants such as bile salts or fusidic acids or other detergents. In addition, if properly formulated in enteric or encapsulated formulations, oral, rectal, intravaginal administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 1 µg/kg/day to 10 mg/kg/day of subject. More preferably, this dose is at least between 0.01 mg/kg/day and 1 mg/kg/day. Variations in the needed dosage are to be expected in view of the nature of the compounds used and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide of the invention, such as a DNA or RNA, to encode a polypeptide *ex vivo,* and for example, by the use of a retroviral plasmid vector. The cells are then introduced into the subject.

The examples below are carried out using standard techniques, which are well known and routine to those of skill in the art, except where otherwise described in detail. The examples illustrate, but do not limit the invention.

### Example 1

### Cloning of HNARA10 cDNA.

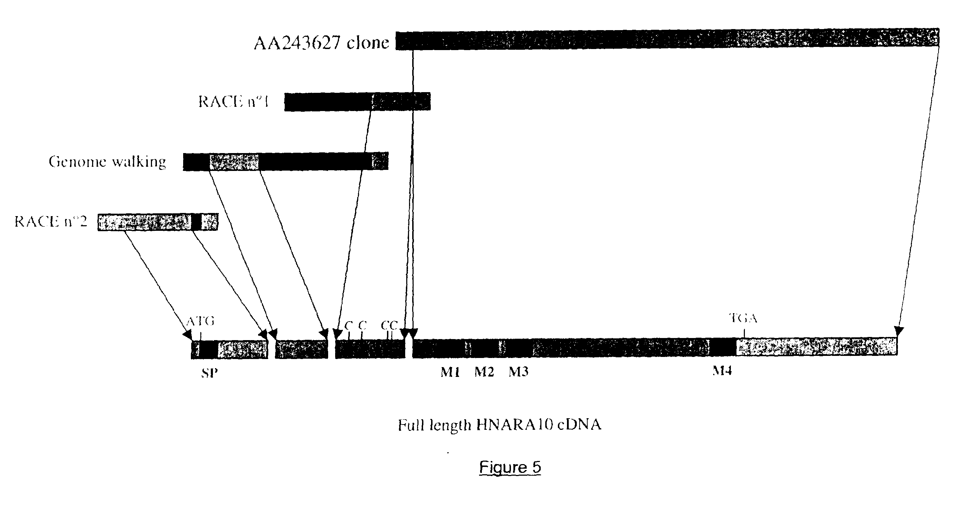

A partial cDNA sequence of HNARA10 was first identified by homology searching using the rat acetylcholine receptor alpha 9 subunit protein sequence (Swissprot accession number P43144) against the GeneBank EST database using tblastn algorithm. One EST (AA243627) from a germinal center B cell cDNA library was found with about 50 % homology, which had been annotated has putative neuronal acetylcholine receptor alpha 9 chain precursor. Complete sequencing of this clone showed that it was truncated at its 5' end and contained part (about 55 %) of the coding sequence of an apparently novel acetylcholine receptor subunit and a 3' untranslated region. The missing 5' coding sequence was obtained by 5' RACE and from human genomic DNA as described in Figure 5. 5' RACE experiments were carried out from the partial cDNA AA243627 clone using human skeletal muscle marathon-ready™ cDNA (Clontech Laboratories, Inc). However, all products obtained showed the presence of an unspliced intron which could not be bypassed by the RACE experiments. The coding sequence situated in 5' of this intron was therefore obtained from human genomic DNA using the Genome Walker system (Clontech). Further 5' RACE amplifications were then carried out from the coding sequence obtained from genomic clones using Marathon-ready™ cDNA from human skeletal muscle which allowed the cloning of the entire coding sequence, although another partially-spliced intron was found in these clones. A full length coding HNARA10 sequence was then assembled by ligating together PCR products generated from the RACE and genomic clones and the original AA243627 clone (Figure 5) into the BamH I -Xho I sites of the expression vector pcDNA3 (Invitrogen Inc). This construct was named ha10pcm7. Sequence of the gene was confirmed by double strand sequencing of at least 3 clones of each RACE or genomic clones described above and by sequencing of the complete cDNA clone after assembly. A blast search of this sequence against GenBank release 98 showed that this gene was indeed completely novel. Similar search against GenBank EST database showed the presence of 11 other EST containing a partial sequence highly similar to HNARA10. None of them contained the 5' coding sequence of the HNARA10 gene corresponding to the first 66 amino acid. Furthermore, all the EST sequences corresponding to the region coding for amino acids 67 to 130 of the HNARA10 protein showed the presence of unspliced introns.

### Example 2

### Tissue distribution of HNARA10 mRNA.

Northern blot and mRNA dot blot analyses were carried out to examine HNARA10 gene expression in human tissues, using methods described by, among others, Sambrook *et al.,* cited above. A cDNA probe produced by digestion of the ha10pcm7 construct with the restriction enzymes Sac I and Xho I and containing part of the 3' non coding end of HNARA10 cDNA was labeled with ³²P using the Megaprime™ labeling system (Amersham Pharmacia Biotech), according to the manufacturer's instructions. After labeling, the probe was purified using a Chroma Spin-100™ column (Clontech Laboratories, Inc), according to the manufacturer's protocol. The purified labeled probe was then used to examine various human tissues for HNARA10 mRNA.

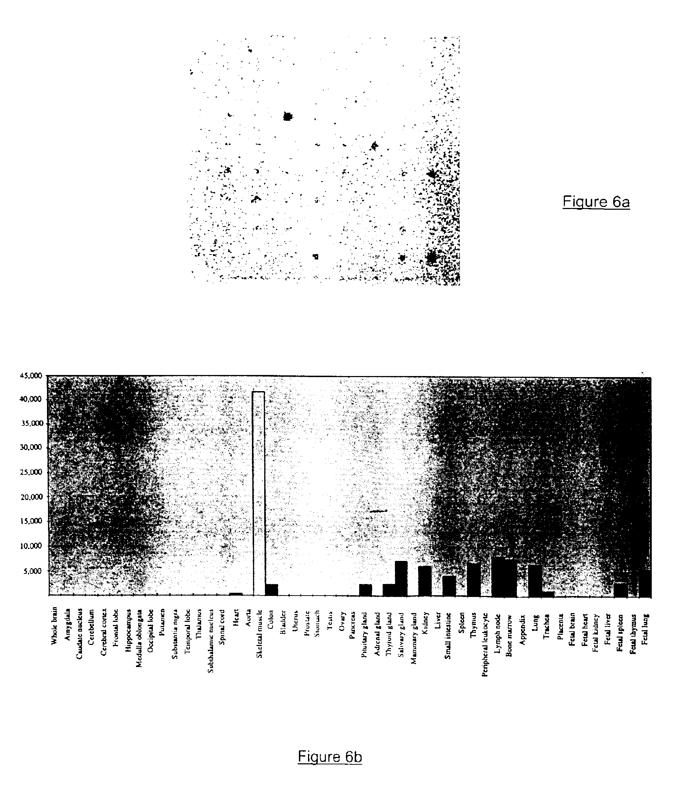

A human RNA Master blot™ (Clontech Laboratories, Inc) containing mRNA from 50 different human tissues was examined with the labeled probe using ExpressHyb™ hybridization solution (Clontech) according to the manufacturer's instructions. Following hybridization and washing, the blot was exposed to a phosphor screen for 48 hours before scanning onto a phosphor imager STORM (Molecular Dynamics) according to the procedures recommended by the manufacturer. The results (Figure 6) show mainly a strong specific expression of HNARA10 mRNA in skeletal muscle. A low level expression, barely above background, was also detected in some other tissues.

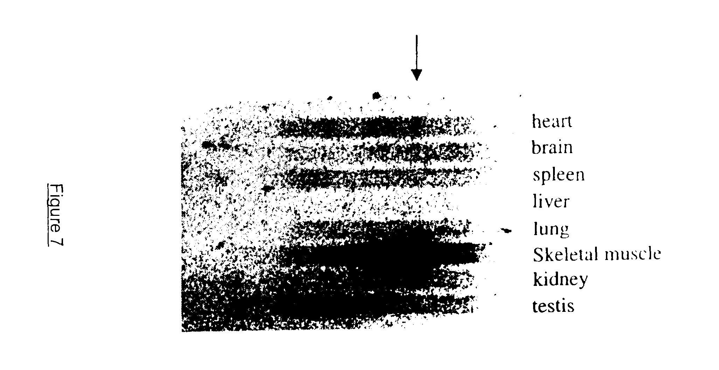

A human Multiple Tissue Northern (MTN) blot (Clontech) containing mRNA from 8 human tissues was also examined with the labeled probe using ExpressHyb™ hybridization solution (Clontech) according to the manufacturer's instructions. Following hybridization and washing, the blot was exposed to a phosphor screen for 96 hours before scanning onto a phosphor imager STORM (Molecular Dynamics) according to the procedures recommended by the manufacturer. The results (Figure 7) confirm the strong expression of HNARA10 mRNA in skeletal muscle in which a 6.4 kb mRNA was labeled. A mRNA of similar size was also detected in heart tissue, although at a much lower level of expression.

### Example 3

### Immunolocalization of HNARA10 protein.

Polyclonal antibodies were generated by immunization of rabbits with two polypeptides corresponding to amino acids 179 to 196 (antibody 2255) and 357 to 374 (antibody 2252) of HNARA10 protein. The sera were tested against HEK 293 cells (ATCC CRL-1573) which do not express nAChRs and TE 691 (ATCC CRL-8805) cells which express muscular nAChRs transiently transfected with h10pcm7 vector containing the full length cDNA HNARA10 sequence. Transfected cells as well as untransfected control cells were fixed onto glass slides, incubated with dilution at 1/1000 of the serum of immunized animals (or with the serum before immunization as control) and with a secondary anti-rabbit antibody coupled to peroxidase (DAKO EnVision™ Systems, DAKO A/S). Labeling was carried out using 3-amino-9-ethylcarbazole (AEC+) chromogenic substrate according to the protocol recommended by the manufacturer (DAKO A/S). The results showed that the polyclonal antibodies 2255 allowed a specific immunodetection of HNARA10 protein expressed in both cell types. No signal was detected in untransfected cells nor in transfected cells treated with the serum extracted before immunization. The absence of signal in untransfected TE 671 cells indicates that the antibodies do not cross react with muscular nAChR subunits. In addition, the antibodies were also tested against PC12 cells (ATCC CRL-1721) which express alpha 3, alpha 5, alpha 7, beta 2 and beta 4 rat nAChR subunits, and against HEK 293 cells transfected with the human alpha 4 and beta 2 nAChR subunits, and no cross reaction was detected, indicating that the polyclonal antibodies 2255 were highly specific. These antibodies were then tested on slices of whole rat embryo at stage E18 (Novagen) for immunohistology. Specific signal was detected in the rat intestine and, to a lower level, in the pancreas, indicating that the antibody 2255 cross-reacted with the rat HNARA10 orthologue protein. Immunolocalization was then carried out on slices from adult rat. The results show a strong specific labeling of cells in the stomach antrum and at the basis of the duodenum, in the pyloric region. The cells labeled appear to be the gastrin-secreting enterochromaffin cells. Immunological analysis of human jejunum slices also showed some scattered labeled cells which could also correspond to gastrin-secreting cells which are present at a low level in this portion of the intestin.

These results suggest that nAChRs containing the HNARA10 subunit are present in gastrin enterochromaffin cells of the digestive system where they may be involved in controlling gastrin secretion.

## Claims

1. An isolated polynucleotide comprising a nucleotide sequence at least 80% identical to a nucleotide sequence selected from the group consisting of:
(a) the nucleotide sequence set forth in SEQ ID NO:1;
(b) a nucleotide sequence encoding the HNARA10 polypeptide set forth in SEQ ID NO:2;
(c) the nucleotide sequence comprising the sequence located between nucleotide numbers 43 to 1392 set forth in SEQ ID NO:1;
(d) the nucleotide sequence encoding a polypeptide comprising amino acids from about 179 to about 196 set forth in SEQ ID NO:2;
(e) the nucleotide sequence encoding a polypeptide comprising amino acids from about 357 to about 374 set forth in SEQ ID NO:2;
(f) a nucleotide sequence encoding the HNARA10 polypeptide expressed by the cDNA clone contained in ATCC deposit number PTA-315;
(g) a nucleotide sequence able to hybridize with the nucleotide sequence set forth in SEQ ID NO:1 or the cDNA clone contained in ATCC deposit number PTA-315;
(h) a nucleotide sequence encoding the mature HNARA10 polypeptide expressed by the cDNA clone contained in ATCC deposit number PTA-315;
(i) a nucleotide sequence complementary to any of the nucleotide sequences defined in (a), (b), (c), (d), (e), (f), (g) or (h) above, as well as fragments, variants, derivatives, mutated forms of these nucleotide sequences.

2. Polynucleotide according to claim 1 which is DNA or RNA.

3. Polynucleotide according to claim 1 or 2, wherein said nucleotide sequence is at least 80% identical to that contained in SEQ ID NO:1.

4. Polynucleotide according to any of claims 1 to 3, which is the polynucleotide of SEQ ID NO: 1.

5. Polynucleotide according to claim 1 useful as a nucleic probe, wherein it comprises the nucleotide sequence located between nucleotide number 998 and nucleotide number 1366, or the nucleotide sequence located between nucleotide number 1396 and nucleotide number 1914 of the sequence set forth in SEQ ID NO:1.

6. A DNA or RNA molecule constituting a recombinant vector, comprising an expression system which is capable of producing a HNARA10 polypeptide when said expression system is present in a compatible host cell, wherein said DNA or RNA molecule comprises a polynucleotide according to any of claims 1 to 4.

7. A host cell containing the expression system of claim 6.

8. A process for producing a HNARA10 polypeptide, comprising the steps of transforming or transfecting a host cell with an expression system according to claim 6, culturing said host cell under appropriate culture conditions so that said host cell produces the HNARA10 polypeptide, and recovering the polypeptide from the culture.

9. An isolated HNARA10 polypeptide comprising an amino acid sequence which is at least 80% identical to a sequence selected from the group consisting of:
(a) the amino acid sequence set forth in SEQ ID NO:2;
(b) the amino acid sequence of the HNARA10 polypeptide having the complete amino acid sequence encoded by the cDNA clone contained in ATCC deposit number PTA-315;
(c) the amino acid sequence of the mature HNARA10 polypeptide having the complete amino acid sequence encoded by the cDNA clone contained in ATCC deposit number PTA-315; as well as fragments, derivatives, variants or mutated forms of these amino acid sequences.

10. Polypeptide according to claim 9, comprising the amino acid sequence of SEQ ID NO:2.

11. Polypeptide according to claim 9 or 10, consisting of the amino acid sequence of SEQ ID NO:2.

12. Polypeptide according to any of claims 9 to 11, obtained by the process of claim 8.

13. Polypeptide according to any of claims 9 to 11, obtained by chemical synthesis.

14. A monoclonal or polyclonal antibody, or a fragment thereof, able to recognize a polypeptide according to any of claims 9 to 11.

15. An antibody according to claim 14, able to recognize a polypeptide comprising amino acid residues from about 84 to about 101, amino acid residues from about 120 to about 158, amino acid residues from about 177 to about 196, amino acid residues from about 207 to about 226, amino acid residues from about 348 to about 395, amino acid residues from about 405 to about 427, amino acid residues from about 179 to about 196 or amino acid residues from about 357 to about 374 of the amino acid sequence set forth in SEQ ID NO:2.

16. A method for identifying agonists of HNARA10 polypeptide comprising :
(a) contacting a host cell according to claim 7 with a candidate compound; and
(b) determining whether the candidate compound effects a signal generated by activation of the HNARA10 polypeptide.

17. An agonist of HNARA10 obtainable by the method of claim 16.

18. An agonist of HNARA10 according to claim 17, wherein the candidate compounds are selected among compounds which present a structural or functional, or both, similarity with known agonists of the nicotinic receptor family, or compounds which are known to be involved in a large number of biological processes.

19. A method for identifying antagonists of HNARA10 polypeptide comprising :
(a) contacting a host cell according to claim 7 with an agonist; and
(b) determining whether the signal generated by said agonist is diminished in the presence of a candidate compound.

20. An antagonist of HNARA10 obtainable by the method of claim 19.

21. An antagonist of HNARA10 according to claim 20, wherein the candidate compounds are selected among compounds which present a structural or functional, or both, similarity with known antagonists of the nicotinic receptor family, or compounds which are known to be involved in a large number of biological processes.

22. A method for the treatment of a subject in need of enhanced activity or expression of HNARA10 polypeptide according to any of claims 9 to 11 comprising :
(a) administering to the subject a therapeutically effective amount of an agonist to said receptor; and/or
(b) administering to the subject a polynucleotide according to any of claims 1 to 4 in a form so as to effect production of said receptor activity *in vivo.*

23. A method for the treatment of a subject having need to alter, diminish or inhibit activity or expression of HNARA10 polypeptide according to any of claims 9 to 11 comprising :
(a) administering to the subject a therapeutically effective amount of an antagonist to said receptor; and/or
(b) administering to the subject a nucleic acid molecule that inhibits the expression of the nucleotide sequence encoding said receptor; and/or
(c) administering to the subject a therapeutically effective amount of a polypeptide that competes with said receptor for its ligand.

24. A process for diagnosing a disease or a susceptibility to a disease in a subject related to expression or activity of HNARA10 polypeptide of any of claims 9 to 11 comprising :
(a) determining the presence or absence of a mutation in the nucleotide sequence encoding said HNARA10 polypeptide in the genome of said subject; and/or
(b) analyzing for the presence or amount of said HNARA10 polypeptide expression in a sample derived from said subject.

25. Pharmaceutical composition, for preventing and/or treating in a subject a disease or susceptibility to a disease related to an under-expression or under-activity of HNARA10, said composition comprising a therapeutically effective amount of at least an agonist of HNARA10 according to claim 18, at least a polypeptide according to any of claims 9 to 11, and/or at least a nucleotide sequence able to express *in vivo* a HNARA10 polypeptide according to any of claims 9 to 11.

26. Pharmaceutical composition for preventing and/or treating in a subject a disease or susceptibility to a disease related to over-activity or over-expression of HNARA10, said composition comprising a therapeutically effective amount of at least an antagonist of HNARA10 according to claim 21, at least a nucleic acid molecule that inhibits the expression of the nucleotide sequence encoding HNARA10 receptor and/or at least a peptide which competes with HNARA10 receptor.

27. Pharmaceutical composition according to claim 25 or 26, comprising a recombinant vector according to claim 6, able to express *in vivo* a polypeptide according to any of claims 9 to 11.

28. Pharmaceutical composition according to any of claims 25 to 27, further comprising at least a pharmaceutically acceptable carrier.

29. Pharmaceutical composition according to any of claims 25 to 28 for prevention and/or treatment of Alzheimer's disease, memory disorder, epilepsy, schizophrenia, chorea, Tourette's syndrome, depression, nicotine dependence, muscular dystrophy, Parkinson's disease, pain, cancer, gastrointestinal ulcer, gastrointestinal motility disorder, endocrinal dysfunction, neuronal degenerative disease, stroke or appetite disorder, or for producing analgesia or anesthesia.
